# EUROPEAN PATENT APPLICATION

(11) **EP 4 588 935 A1**
(43) Date of publication of application: **23.07.2025**
(21) Application number: 22965680.6
(22) Date of filing: 30.12.2022
(51) Int. Cl.: C07K 14/47, C07K 16/18, C12N 5/20, C12N 15/13, G01N 33/68, G01N 33/569, G01N 33/577, A61K 45/00, A61K 39/395, A61P 29/00, A61P 31/04, A61P 31/12

(54) **MARKER AND METHOD FOR DETECTING INFLAMMATION-RELATED DISEASES**

(30) Priority: 16.11.2022 CN 202211460978
(71) Applicant: Beijing MDTK Biotechnology Co. Ltd, Beijing 102206 (CN)
(72) Inventor: WANG, Jianxia, Beijing 102206 (CN); LI, Jun, Beijing 102206 (CN); LIU, Ying, Beijing 102206 (CN); KANG, Haoxiang, Beijing 102206 (CN); MAO, Sufang, Beijing 102206 (CN); CAO, Yu, Beijing 102206 (CN)
(74) Representative: Huang, Liwei
(86) International application number: PCT/CN2022/144118
(87) International publication number: WO 2024/103504

(57) **Abstract**

The present application relates to a marker and a method for detecting inflammation-related diseases. The marker of the present application can stably exist in an ex vivo body fluid sample and does not gather on a cell membrane, so the detection result is very accurate. The marker can be widely applied to the medical detection of inflammation-related diseases, having good clinical application prospects.

## Description

### Technical Field

The present application relates to a marker and a method for detecting inflammation-related diseases and falls within the field of biotechnology.

### Background Art

Inflammation, a body's defense response to stimuli, manifests as redness, swelling, heat, pain, and dysfunction, which may or may not be caused by infection. Among them, inflammation caused by infection, such as infectious diseases, whether of bacterial, viral, or other origin, poses acute and chronic challenges to human health. Viruses include DNA viruses and RNA viruses. Bacteria include gram-positive and gram-negative bacteria and may include mycoplasmas (bacteria lacking cell walls). In addition to pathogenic bacteria, other microorganisms such as yeasts, fungi, and other small pathogenic organisms can also cause some infectious diseases.

Currently, many detection markers are used to detect inflammation-related diseases (such as sepsis) in clinic, but these detection markers lack specificity for infection: for example, CRP is an acute phase response protein that is synthesized and secreted by the liver in the serum of patients in some pathological conditions, but in addition to bacterial infection, viral infection, acute rejection, cardiovascular system diseases, and surgery can cause the increase of CRP, so CRP lacks specificity for infection, and the increase of CRP concentration is not related to the clinical prognosis; other markers used clinically, such as PCT, IL-6, SAA, etc. also share similar deficiencies, leading to the limited usefulness of existing markers for the diagnosis or treatment of inflammation-related diseases, including sepsis.

### Summary of the Invention

In view of the technical problem as described above, the present application provides, in one aspect, a marker for inflammation-related diseases, the marker is GSDMD-CT or a fragment thereof, a modified variant of GSDMD-CT or a fragment thereof, and/or a derivative of GSDMD-CT or a fragment thereof. In one embodiment, the sequence of the GSDMD-CT fragment is as shown in SEQ ID NO. 4. In one embodiment, the sequence of GSDMD-CT is as shown in SEQ ID NO. 3. In a second aspect, the present application provides a nucleic acid encoding a marker as described above. In a third aspect, the present application provides a biological material including a marker as described above, or a nucleic acid encoding the same, the material is one or more of an expression cassette, a recombinant vector, a recombinant microorganism, or a transgenic cell line. In a fourth aspect, the present application provides a ligand that binds to a marker as described above, a nucleic acid as described above, and/or a biological material as described above.

In one embodiment, the ligand includes a nucleic acid aptamer, a peptide aptamer, a peptibody, a mimetic, a phage, an inhibitor, a compound, and/or an antibody. In one embodiment, the antibody is a polyclonal antibody, including a Nanobody, a monospecific antibody, and/or a polyclonal antibody. In one embodiment, the ligand bears a recognizable label; Preferably, the label may be a dye, an epitope tag, a fluorescent moiety, a luminescent moiety, a chemiluminescent moiety, an enzyme label, a magnetic label, a paramagnetic label, a contrast agent, a nanoparticle, a radioisotope, biotin, streptavidin, and a quencher.

The present application also provides a kit or composition including a marker as previously described, a nucleic acid as previously described, a biological material as previously described, and/or a ligand as previously described. In one embodiment, the kit or composition further includes a ligand that binds to other markers, and/or reagents for qualitative or quantitative detection assays. In one embodiment, the qualitative or quantitative detection is one or more of a nucleic acid detection, an immunodetection, a chemical detection, and/or a cell count detection. In one embodiment, the additional markers include one or more of GSDMD-CN, GSDMD-NT, PCT, IL-6, SAA, CRP, or IL-1β. In one embodiment, the kit or composition further includes a preparation or drug for use as a standard and for calibration. In one embodiment, the preparations or drugs used as standards and for calibration include GSDMD-CN, GSDMD-NT, GSDMD-CT, or antibodies thereto.

The present application also provides a system or device for determining or inhibiting the degree of pyroptosis, preventing or diagnosing inflammation-related diseases, treating inflammation-related diseases, assessing the severity of inflammation-related diseases or the prognosis of a patient thereof, or monitoring the progression of inflammation-related diseases, the system or device including the use of the markers as previously described, the nucleic acids as previously described, the biological materials as previously described, the ligands as previously described, and/or the kits or compositions as previously described.

The present application provides the use of a marker as previously described, a nucleic acid as previously described, a biological material as previously described, a ligand as previously described, and/or a kit or composition as previously described, in the manufacture of a drug, compound, composition or preparation for determining or inhibiting the degree of pyroptosis, for preventing or diagnosing inflammation-related diseases, for treating inflammation-related diseases, for assessing the severity of inflammation-related diseases or the prognosis of a patient thereof, or for monitoring the progression of inflammation-related diseases.

The present application provides the use of a marker as previously described, a nucleic acid as previously described, a biological material as previously described, a ligand as previously described, and/or a kit or composition as previously described, to determine or inhibit the degree of pyroptosis, to prevent or diagnose inflammation-related diseases, to treat inflammation-related diseases, to assess the severity of inflammation-related diseases or the prognosis of a patient thereof, or to monitor the progression of inflammation-related diseases.

The present application provides a method of determining or inhibiting the degree of pyroptosis, preventing or diagnosing inflammation-related diseases, treating inflammation-related diseases, assessing the severity of inflammation-related diseases or a prognosis of a patient thereof, or monitoring the progression of inflammation-related diseases, the method including qualitatively or quantitatively detecting in a sample a marker that is GSDMD-CT or a fragment thereof, a modified variant of GSDMD-CT or a fragment thereof, and/or a derivative of GSDMD-CT or a fragment thereof, or a nucleic acid encoding the marker. In one embodiment, the detection employs a ligand as described above, and/or a kit as previously described. In one embodiment, the sample is selected from the group consisting of human or animal serum, plasma, urine, blood, or cerebrospinal fluid, and may also be a cell culture fluid, cell lysate.

The present application also provides a method of screening for a drug, compound, composition, or preparation for detecting or inhibiting the degree of pyroptosis, preventing or diagnosing or treating inflammation-related diseases, assessing the severity thereof or a prognosis of a patient thereof, or monitoring the progression of inflammation-related diseases, the method including using the drug, compound, composition or preparation to specifically identify the marker as previously described or nucleic acid encoding the same, and/or the biological material as previously described.

In one embodiment, the drug, compound, composition, or preparation includes a ligand that binds to a marker as previously described, a nucleic acid as previously described, and/or a biological material as previously described. In one embodiment, the ligand includes a nucleic acid aptamer, a peptide aptamer, a peptibody, a mimetic, a phage, an inhibitor, and/or an antibody. In one embodiment, the antibody includes a Nanobody, a monospecific antibody, and/or a polyclonal antibody.

In one embodiment, the inflammation-related diseases include diseases caused by pyroptosis or diseases accompanied by cellular inflammatory necrosis associated with pyroptosis; preferably, the inflammation-related diseases are: inflammatory diseases caused by infection, especially inflammation caused by bacterial infection, inflammation caused by viral infection, such as pneumonia, peritonitis, cholangitis, urinary system infection, cellulitis, meningitis, abscess and the like and complications of infection thereof, surgery, trauma, multiple trauma, burn, septicemia, sepsis, septic shock and the like; autoimmune diseases such as arthritis, gout, scleroderma, and the like; chronic diseases such as diabetes, chronic obstructive bronchial disease, leukemia, aplastic anemia, urinary calculi and the like; other diseases such as one or more of cancer, diabetes, kidney disease, senile dementia and the like.

The present application also relates to compositions of GSDMD-CT, and/or fragments thereof, and/or modified variants thereof, and/or derivatives thereof, in combination with other markers, and to the use of these substances in medicine, in particular in the diagnosis or treatment of inflammation-related diseases, or in the screening of drugs for the prevention, diagnosis or treatment of inflammation-related diseases.

In embodiments, the present application also provides systems, methods, and devices for testing for the presence of one or more of a plurality of markers in a plurality or a single small-volume clinical sample, or an aliquot thereof. In an embodiment, the system, method, or device is a POS system, method, or device. The marker may be indicative of an inflammatory disease; in certain embodiments, the marker may be indicative of an infectious disease; in certain embodiments, the marker may be indicative of sepsis.

In some embodiments, the markers of the present application are used, alone or in combination with other markers, to (i) detect and quantify the level of one or more proteins or nucleic acids in a sample, (ii) compare the quantified level to a control level of proteins or nucleic acids in a control population, and (iii) decide whether an individual to whom the sample corresponds has inflammation-related diseases, or has a risk or degree of having inflammation-related diseases, as compared to the control population.

### Brief Description of the Drawings

Various other advantages and benefits will become apparent to those of ordinary skill in the art upon reading the following detailed description of the preferred embodiments. The drawings are only for the purposes of illustrating the preferred embodiments and are not to be construed as limiting the present application.
FIG. 1 shows a schematic representation of the use of an antibody that specifically binds to the N-terminal amino acid sequence of GSDMD-CT.
FIG. 2 shows a schematic representation of the use of an antibody that specifically binds to the N-terminal amino acid sequence of GSDMD-CT together with antibodies directed against other portions or fragments of GSDMD-CT.
FIG. 3 shows a schematic representation of the use of an antibody directed against the non-N-terminal amino acid sequence of GSDMD-CT.
FIG. 4 shows that the antibodies numbered #25, #44 and #50 in Table 1 specifically react with the N-terminal amino acid sequence of GSDMD-CT.
FIG. 5 shows competitive inhibition experiments for antibodies numbered #25, #44, and #50 in Table 1.
FIG. 6 shows a standard curve for the GSDMD-CT chemiluminescent detection system.
FIG. 7 shows the expression levels of GSDMD-CT in HC, non-SIRS, and inflammation.
FIG. 8 shows the expression levels of GSDMD-CT in HC, non-SIRS, inflammation, and infectious inflammation.
FIG. 9 shows the diagnostic efficacy ROC of GSDMD-CT, PCT, and CRP for inflammation (SIRS).
FIG. 10 shows the diagnostic efficacy ROC of GSDMD-CT, PCT, and CRP for Sepsis.
FIG. 11 shows a positive correlation between PCT and GSDMD-CT expression.
FIG. 12 shows a weak correlation between CRP and GSDMD-CT expression.
FIG. 13 shows the use of different markers to continuously monitor infection in a single subject.
FIG. 14 shows a comparison of GSDMD-CT concentration content in samples from patients infected with Gram-negative bacteria and Gram-positive bacteria.
FIG. 15 shows specific monoclonal antibodies prepared from the antigen of SEQ ID NO. 4.

### Detailed Description of the Invention

The following examples are provided only to more clearly illustrate the technical solution of the present application, and are therefore given by way of illustration only and should not be construed to limit the scope of the present application.

Unless defined otherwise, all technical and scientific terms used herein have the same meaning as commonly understood by one of ordinary skill in the art to which the present application belongs; The terminology used herein is to describe particular embodiments only and is not intended to be limiting of the present application; The terms "comprise" and "include", and any variation thereof, in the description and claims of the present application and the description of the figures as described above are intended to cover a non-exclusive inclusion.

Reference herein to "embodiment" means that a particular feature, structure, or characteristic described in connection with the embodiment may be included in at least one embodiment of the present application. The appearances of the phrase in various places in the specification are not necessarily all referring to the same embodiment, nor are separate or alternative embodiments mutually exclusive of other embodiments. It is to be expressly and implicitly understood by those skilled in the art that the embodiments described herein may be combined with other embodiments.

In the description of the embodiments of the present application, the term "and/or" is merely an association relationship describing an associated object, meaning that there may be three relationships, for example, A and/or B, which may mean that: There are three cases of A alone, A and B together, and B alone. In addition, the character "/", as used herein, generally indicates the context in which the associated object is an "or".

Reference herein to "one or more" or "at least one" means that at least one of the elements is present; there may be a plurality of such elements unless specifically limited otherwise.

As used in the specification and the appended claims, the singular forms "a", "an", and "the" include plural referents unless the context clearly dictates otherwise.

As used herein, the term "pyroptosis" refers to the cleavage of GSDMD (GSDMD-CT) protein by enzymes such as caspase -1/4/5/11, etc. under the stimulation of activated inflammatory bodies, intracytoplasmic lipopolysaccharides, etc. to generate N-terminal and C-terminal residues, wherein the N-terminal is capable of forming an oligomer, forming a transmembrane pore in the cell membrane, and inducing inflammatory death of the cell, i.e. pyroptosis.

In the context of medicine, "diagnosis" is the act or process of identifying one or more health conditions, including disease and/or injury, by evaluating one or more factors, which may include a patient's medical history, physical examination, review of symptoms, and review of data from one or more laboratory tests, to identify or determine the nature and/or cause of the disease. In the present application, if not defined otherwise, they include not only diagnoses in the sense of identifying a particular disease, but also screening of asymptomatic or high-risk populations at risk for a disease or suspected of having a disease, or monitoring of untreated or treated patients, as well as monitoring of the course of treatment and making early predictions and/or survival predictions.

As used herein, the term "treat" refers to reducing or alleviating the progression, severity, and/or duration of a disease onset and/or symptoms.

As used herein, the term "prevent" refers to reducing the occurrence of a disorder or condition in a treated sample relative to an untreated control sample, or delaying the occurrence of one or more symptoms of a disorder or condition relative to an untreated control sample, or reducing the severity of one or more symptoms of a disorder or condition.

As used herein, "monitor" refers to tracking those diseases, conditions, complications, or risks that have been diagnosed, e.g. analyzing the progress of the disease, or analyzing the effect of a particular treatment on the progress of the disease or condition.

As used herein, "assess" refers to prejudging the condition or complication of a disease before the symptoms or markers of the disease become apparent, or have significantly changed.

As used herein, a "patient", "individual" or "subject" refers to a human or animal.

"Inflammation-related disease" refers to an inflammatory disease, or a condition with inflammatory necrosis of cells resulting from pyroptosis, including, but not limited to: inflammatory diseases caused by infection, especially inflammation caused by bacterial infection, inflammation caused by viral infection, such as pneumonia, peritonitis, cholangitis, urinary system infection, cellulitis, meningitis, abscess and the like and complications of infection thereof, surgery, multiple trauma, trauma, burn, septicemia, sepsis, septic shock and the like; autoimmune diseases such as arthritis, gout, scleroderma, and the like; chronic diseases such as diabetes, chronic obstructive bronchial disease, leukemia, aplastic anemia, urinary calculi and the like; other diseases such as one or more of cancer, diabetes, kidney disease, senile dementia and the like.

"Infection" within the scope of the present application refers to a pathological process caused by the invasion of normal sterile tissues or body fluids by potential pathogenic agents/pathogens, organisms, and/or microorganisms, such as the infection of fungi, bacteria, viruses and/or parasites. The infection may be a local or systemic infection. Furthermore, a subject suffering from an infection may suffer from more than one source of infection at the same time. For example, the subject may suffer from viral infections and fungal infections; Bacterial and fungal infections, as well as bacterial, fungal, and viral infections.

As used herein, the term "control population" or "control sample" can be a negative control population or a positive control population. In some embodiments, the control population is a population of individuals with the same disease as the subject individual. In some embodiments, the control population is a population of individuals with the same disease as the subject individual and not developing a crisis response and/or life-threatening response to the disease. In some embodiments, the control population is a population of normal individuals. In some embodiments, the control population is a population of individuals in which a majority of the members of the control population do not have the same disease as the subject individual. In some embodiments, the population as described above is an unbiased population.

A "marker" is not limited to a form, and maybe a biomarker or a body fluid marker, and maybe a protein, a polypeptide, or an antibody.

"GSDMD-CN" or "full-length GSDMD" refers to an intact GSDMD (Gasdermin D) protein having both an amino terminus and a carboxy terminus. The present application also relates to modified variants of the entire GSDMD protein and/or derivatives of the entire GSDMD protein. A "GSDMD-CN fragment" refers to a polypeptide that is contained in "GSDMD-CN", but may not be "GSDMD-NT" or "GSDMD-CT". The present application also relates to modified variants of the "GSDMD-CN fragment" and/or derivatives thereof.

As used herein, "GSDMD-NT" refers to the N-terminal of GSDMD, which has perforating activity and is capable of forming aggregates on the cell membrane, causing subsequent cell volume expansion and blebbing of the cell membrane to the outside of the cell, which ultimately leads to pyroptosis.

As used herein, "GSDMD-CT" refers to the C-terminus of GSDMD, which is the C-terminal domain of GSDMD released after cleavage by an enzyme such as caspase -1/4/5/11, e.g. as shown in SEQ ID NO. 3, or an amino acid sequence having more than 50%-60% homology, more than 61%-70% homology, or more than 71%-80% homology, or more than 81%-90% homology, or more than 91%-99% homology, or 100% homology to SEQ ID NO. 3. Preferably, the carboxyl terminus of the GSDMD characterized by these sequences shares immunoreactivity with the "GSDMD-CT" characterized by SEQ ID NO. 3. The protocol of the present application is also applicable to the detection of modified variants of "GSDMD-CT" and/or derivatives thereof.

A "GSDMD-CT fragment" is a polypeptide that is contained in "GSDMD-CT". A "GSDMD-CT fragment" includes amino acids from the corresponding position of the amino acid at position 1 to the corresponding position of the amino acid at position N of SEQ ID NO. 3, such as the amino acid fragment as shown in SEQ ID NO. 4; or an amino acid sequence having more than 50%-60% homology, more than 61%-70% homology, or more than 71%-80% homology, or more than 81%-90% homology, or more than 91%-99% homology, or 100% homology to the amino acid sequence of "GSDMD-CT fragment" as previously described in this paragraph, preferably, the carboxyl terminus of the GSDMD characterized by these sequences has a common immunoreactivity with the "GSDMD-CT fragment" as previously described. The protocol of the present application is also applicable to the detection of modified variants of the "GSDMD-CT fragment" and/or derivatives thereof.

As used herein, the term "immunodetection" or "immunoassay" refers to the detection, identification, characterization, or quantification of an amino acid target (which may be a small peptide, polypeptide, protein, or protein macromolecule) in a sample by immunological principles. Immunodetection includes, for example, direct or competitive binding assays using techniques such as radioimmunoassays, enzyme-linked immunosorbent assays (ELISA), "sandwich" immunoassays, immunoprecipitation assays, fluorescent immunoassays, and protein A immunoassays. Immunodetection typically uses antibodies or antibody fragments, but binding proteins or carrier proteins that bind target molecules with high specificity can also be used. In one embodiment, the immunoassay is a solid phase immunoassay, e.g. a heterogeneous immunoassay using an immobilized first specific binding agent and a second solubilized specific binding agent that carries a detectable marker or that can be selectively labeled by reaction with a labeled marker molecule. Preferably, the immunoassay is a homogeneous immunoassay.

As used herein, a "sample", "biological sample" or "clinical sample" refers to a sample of fluid, tissue, secretion, or fecal matter obtained from a subject, including, but not limited to, blood, serum, plasma, saliva, sputum, urine, gastric fluid, digestive fluid, tears, sweat, feces, semen, vaginal fluid, interstitial fluid, a fluid derived from tumor tissue, intraocular fluid, mucus, cerumen, oil, glandular secretions, spinal fluid, skin, cerebrospinal fluid from within the skull; tissue, fluid or substance, biopsy fluid or material from a nasal swab, throat swab, mouth swab (e.g. buccal swab), vaginal swab, or nasopharyngeal wash; fetal fluid, amniotic fluid, umbilical cord blood, lymph fluid, luminal fluid, pus, a microflora obtained from a subject, meconium, milk, or other secretions or excretions.

As used herein, the term "ligand" means capable of selectively binding to a marker, including, but not limited to, a nucleic acid aptamer, peptide aptamer, peptibody, mimetic, bacteriophage, inhibitor, compound, antibody, and the like.

The term "antibody" encompasses monoclonal antibodies and polyclonal antibodies and also encompasses antigen-binding fragments of antibodies. The term "antigen-binding fragment of an antibody" (or simply "antibody portion" or "antibody fragment") refers to one or more fragments of a full-length antibody that retain the ability to specifically bind to a marker of the present application. Examples of "antigen-binding fragments" include (i) the Fab fragment, a monovalent fragment consisting of the VL, VH, CL and CH1 domains; (ii) an F(ab') 2 fragment, a bivalent fragment including two Fab fragments linked by a disulfide bridge at the hinge region; (iii) a Fd fragment consisting of the VH and CH1 domains; (iv) Fv fragments consisting of the VL and VH domains of a single arm of an antibody; (v) a dAb fragment consisting of a VH domain; and (vi) an isolated complementarity determining region (CDR). In addition, while the two domains of the Fv fragment, VL and VH, are encoded by separate genes, they can be joined, using recombinant methods, by a synthetic linker that enables the two domains to be produced as a single protein chain in which the VL and VH regions paired to form a monovalent molecule, known as a single chain Fv (scFv). Such single-chain antibodies are also intended to be encompassed by the term "antigen-binding fragment of an antibody". These antibody fragments are obtained using conventional techniques known to those skilled in the art, and the fragments are screened in the same manner as an intact antibody. An antibody can be monospecific, e.g. a monoclonal antibody or an antigen-binding fragment thereof. The term "monospecific" refers to exhibiting a single binding specificity and affinity for a particular target (e.g. epitope). Thus, a "monospecific antibody" includes a "monoclonal antibody" or a "monoclonal antibody composition", which, as used herein, refers to a preparation of antibodies or fragments thereof of a single molecule composition.

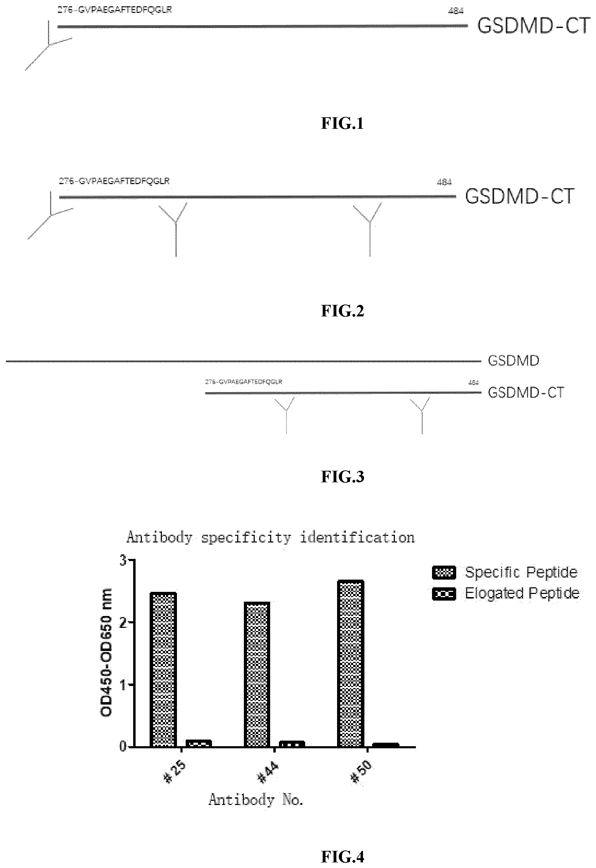

In some embodiments, an antibody of the present application specifically binds to the N-terminal amino acid sequence of GSDMD-CT, e.g. polypeptide number #1, i.e. SEQ ID NO. 4: GVPAEGAFTEDFQGLR (as shown in FIG. 1). Such antibodies can be used to establish competitive inhibition kits that can be used to detect GSDMD-CT concentrations by immunological competitive inhibition assays. Alternatively, such antibodies may be combined with antibodies directed against other portions or fragments of GSDMD-CT (as shown in FIG. 2) to form an immunological sandwich kit that specifically detects GSDMD-CT, but does not react with GSDMD-NT and fragments thereof or GSDMD-CN (full-length GSDMD). Since the concentration of GSDMD-CT is positively correlated with the degree of pyroptosis, it can be used to assess the degree of pyroptosis or inflammation.

In some embodiments, an antibody of the present application is directed against an amino acid sequence other than the N-terminal of GSDMD-CT (e.g. other GSDMD-CT portions or fragments other than SEQ ID NO. 4: GVPAEGAFTEDFQGLR) (as shown in FIG. 3), the antibodies can also be paired with each other to detect GSDMD-CT, as well as GSDMD-CN (full-length GSDMD).

The term "specifically recognize", "selectively bind" or "specifically bind" is when the binding ligand is present in a mixture with other molecules or organisms, and the antibody also preferentially binds or recognizes the binding ligand. The binding may be mediated via covalent or non-covalent interactions or a combination of both.

It is an advantage of the present invention that, on the one hand, GSDMD-CT or a fragment thereof can be released extracellularly, e.g. into the blood, through a small hole in the cell membrane; On the other hand, ex vivo GSDMD-CT or a fragment thereof had an extra-long-term stability in liquid samples-even after storage at room temperature for 2 days, the GSDMD-CT content in plasma and serum remained quite stable (Table 3). Therefore, GSDMD-CT or a fragment thereof is very suitable for in vitro determination of ex vivo liquid samples, i.e. the use of GSDMD-CT or a fragment thereof as a marker to indicate pyroptosis or the degree of pyroptosis is not only accurate but also suitable for detection of ex vivo samples in practical applications.

In order to show the technical idea of the inventors in detail, the present application as a whole provides a marker of inflammation-related diseases, and the use of the marker and substances related thereto in medical detection and drug screening. The markers of the present application are GSDMD-CT or fragments thereof, modified variants of GSDMD-CT or fragments thereof, and/or derivatives of GSDMD-CT or fragments thereof. The markers of the present application are used in the context of in vitro cell experiments, animal experiments, human clinical trials, and the like. The present application further relates to optimized antibodies against GSDMD-CT or against fragments of GSDMD-CT. In a preferred embodiment of the present application, a protein of the amino acid sequence of human GSDMD-CT or a fragment thereof is selected as an immunogen, also for calibration purposes and as a standard solution for preparation.

In a preferred embodiment of the present application, the DNA sequence of human GSDMD-CT or a fragment thereof is artificially synthesized by codon optimization, then constructed into a plasmid, artificially recombinantly expressed, and purified. They were subjected to purity and molecular weight control by SDS-PAGE and lyophilized into aliquots. In a preferred embodiment, the recombinant antigenic protein expressed by the present application is used to produce an antigen, which is injected into an animal to produce antibodies directed against human GSDMD-CT or fragments thereof. Different methods may be used to achieve this goal as known to those skilled in the art. In a preferred embodiment, recombinant GSDMD-CT or a fragment thereof is prokaryotic expressed in E. coli, and the expressed purification tag is excised so that the final expression product has the same amino acid sequence as the native GSDMD-CT or a fragment thereof. Antibodies against the recombinant GSDMD-CT or fragments thereof as described above are produced in mice. In a preferred embodiment of the present application, a mouse monoclonal antibody against the recombinant GSDMD-CT or a fragment thereof as described above is fused, screened, and purified using conventional monoclonal antibody preparation methods. In preferred embodiments, the antibody is provided with a label capable of being detected, e.g. a fluorescent label, a chemiluminescent label, etc.

In a preferred embodiment, a polypeptide fragment (SEQ ID NO. 4) synthesized by the method of polypeptide synthesis, in which a cysteine residue is added at the C-terminal of the polypeptide, is conjugated to KLH, or OVA, or BSA by the method of SMCC to prepare an immunogen, and the animal is immunized; Alternatively, the polypeptide fragments may be conjugated by the method of EDC, KLH, or OVA, or BSA, to produce an immunogen, which immunizes the animal. Antibodies were raised in mice against the polypeptide fragment (SEQ ID NO. 4) as described above as well as epitope fragments within the polypeptide fragment. In a preferred embodiment of the present application, the polypeptide fragment or an epitope fragment within a fragment thereof as described above is preferably: an antibody directed against the polypeptide fragment SEQ ID NO. 4 which is non-reactive with the polypeptide fragment SEQ ID NO. 5. The mouse monoclonal antibody is fused, screened and purified by the traditional monoclonal antibody preparation method. In preferred embodiments, the antibody is provided with a label capable of being detected, e.g. a fluorescent label, a chemiluminescent label, etc.

In a preferred embodiment, the present application relates to the use of the antibodies produced for the detection of the markers of the present application, in particular GSDMD-CT or a fragment thereof, in body fluids and or cell culture fluid samples, as well as the use of kits including one or more antibodies for the detection of the markers of the present application using different assays known to the person skilled in the art. Methods for detecting the binding of antibodies to individual molecules are also known to those of skill in the art. All such known assay formats can be used within the scope of the marker in the present application, e.g. GSDMD-CT or fragment thereof assay. For example, it is within the scope of the present application to determine through a rapid test device, such as a rapid test device for immunochromatography, the so-called POCT test. In a preferred embodiment, ELISA, a turbidimetric inhibition immunoassay device, is used. In a more preferred embodiment, rapid and accurate detection can be achieved using a chemiluminescent assay device. Preferred embodiments of the present application disclose the use of immunodetection or analysis of antibodies generated against the recombinant GSDMD-CT or a fragment thereof as described above. Another preferred embodiment of the present application discloses the use of such antibodies for detecting the concentration of the markers of the present application, in particular GSDMD-CT or fragments thereof, in various body fluids and other biological materials. In a preferred embodiment, the recombinant GSDMD-CT or a fragment thereof can be detected at a concentration of more than 10 pg/ml in body fluid.

Preferred embodiments of the present application disclose the use of antibodies raised against GSDMD-CT or a fragment thereof for detecting the content of GSDMD-CT or a fragment thereof in healthy individuals, individuals infected with inflammation, and/or individuals with sepsis, for determining the severity of pyroptosis in such individuals, and for characterizing a positive correlation between inflammation severity and pyroptosis. Preferred embodiments may also detect a marker of the present application in body fluid, in particular to detect the presence and stability of GSDMD-C or a fragment thereof, and to compare the difference in the concentration of GSDMD-CT or a fragment thereof in a healthy control individual to a patient suffering from one or more inflammation-related diseases. The present application further discloses a significant change in the concentration of a humoral marker, in particular GSDMD-CT or a fragment thereof, in body fluid in inflammation-related diseases state. At least one patient sample is assayed in the present application. In some embodiments, the assay is performed using an automated analytical device or a diagnostic assay. In some embodiments, the assay is performed by a rapid assay, particularly using a single-parameter or multi-parameter assay.

### Example 1 Expression and synthesis of recombinant GSDMD-CT or a fragment thereof

The DNA sequence of human GSDMD-CT or fragments thereof (e.g. SEQ ID NO. 3, etc.) was codon-optimized, artificially synthesized according to conventional methods in the art, then constructed into a plasmid, artificially recombinantly expressed, purified; after removing the purification tags, the final expression product was completely identical to the sequence of natural GSDMD-CT or fragments thereof. Through SDS-PAGE, they were subjected to purity and molecular weight control, concentration determination, and lyophilized into aliquots, both as immunogens and standards. Alternatively, a polypeptide fragment (such as SEQ ID NO. 4 or SEQ ID NO. 5, etc.) synthesized by a polypeptide synthesis method according to a conventional method in the art, a cysteine was added at the C-terminal of the polypeptide during synthesis, and conjugated to KLH, or OVA, or BSA by the method of SMCC, so as to prepare an immunogen, and immunizing animals; or the polypeptide fragment (SEQ ID NO. 4) could be conjugated to KLH, or OVA, or BSA by the method of EDC to produce an immunogen.

### Example 2 Animal immunization

Mice were immunized according to conventional methods in the art. Mice were immunized with complete adjuvant and appropriate antigen for the first time, and then mice were immunized with incomplete adjuvant and appropriate antigen every other week. In the present application, after the 3^{rd} and 4^{th} immunizations, blood was taken from the orbit of the mice, and anti-serum was obtained through centrifugation. The antibody titer of the mouse serum was measured. The mice with the best titer were selected for cell fusion experiments to prepare monoclonal antibodies.

### Example 3 Screening of monoclonal antibodies

### 1. Preparation process

Mice were immunized intraperitoneally with the immunogen at a dose of 50 ug/mouse 3 days prior to cell fusion. After shock immunization, the spleen was fused with Sp2/0 cells, and monoclonal hybridomas were selected. Specifically, the cultured mouse myeloma cells Sp2/0 were collected into a 50 ml centrifuge tube. After the fusion mice were killed by exsanguination, the mice were disinfected systemically by soaking them in a beaker of 75% ethanol. The spleens were then removed from the mice by cutting the lower edge of the left ribs with high-pressure ophthalmic scissors. The spleens were then crushed using a tissue grinder and centrifuged at 1500 rpm for 10 min using a centrifuge. The mouse thymus was then removed with an ophthalmic scissors. The mouse thymus tissue was also crushed with a tissue grinder. The crushed mouse thymus tissue was added to a 15ml centrifuge tube with a sample applicator, followed by adding 2 ml HAT and 1 ml HT to mix well. Splenic cells and Sp2/0 cells were mixed in a ratio of 1:3 to 10, then the centrifuge tube was placed in a water bath at 37°C, and 1 ml of polyethylene glycol (PEG) was slowly added within 1 min. The mixture was allowed to stand in the water bath for 1 min and slowly replenish serum-free IMDM medium to 10 ml. After mixing, the mixture was centrifuged at 1000 rpm/min for 10 min. Then, 10 ml of calf serum was added, the cells were resuspended, and 5 ml of Clone Easy medium, 5 ml of thymocyte mixture (2 ml thymus, 2 ml HAT, 1 ml HT), and 25 ml of semi-solid medium were poured, and mixed well. After mixing well, the suspension was poured into about thirty 3 cm cell culture dishes and placed in a 37°C, 5% CO₂ incubator for culture. After a colony of monoclonal cells was grown, the clones were transferred to a 96-well plate for expansion culture. 200 ul of IMDM cell culture medium containing 20% calf serum, 1×HT, and 10% Clone Easy medium was added to a 96-well cell culture plate, and then the single cell population as described above was picked and added into the wells, placed in a 37°C, 5% CO₂ incubator for culture. Finally, 102 monoclonal antibodies were obtained (see Table 1-2).

**Table 1: MD2106-#5 (78 monoclonal antibodies)**

| | | | | | | | | | |
|---|---|---|---|---|---|---|---|---|---|
| #1 | #11 | #19 | #27 | #35 | #43 | #51 | #59 | #67 | #75 |
| #2 | #12 | #20 | #28 | #36 | #44 | #52 | #60 | #68 | #76 |
| #3 | #13 | #21 | #29 | #37 | #45 | #53 | #61 | #69 | #77 |
| #4 | #14 | #22 | #30 | #38 | #46 | #54 | #62 | #70 | #78 |
| #5 | #15 | #23 | #31 | #39 | #47 | #55 | #63 | #71 | #80 |
| #7 | #16 | #24 | #32 | #40 | #48 | #56 | #64 | #72 | #81 |
| #9 | #17 | #25 | #33 | #41 | #49 | #57 | #65 | #73 | |
| #10 | #18 | #26 | #34 | #42 | #50 | #58 | #66 | #74 | |

**Table 2: MD2025-#4 (34 monoclonal antibodies)**

| | | | | |
|---|---|---|---|---|
| #1 | #10 | #17 | #24 | #31 |
| #2 | #11 | #18 | #25 | #32 |
| #4 | #12 | #19 | #26 | #33 |
| #5 | #13 | #20 | #27 | #34 |
| #6 | #14 | #21 | #28 | #35 |
| #8 | #15 | #22 | #29 | #36 |
| #9 | #16 | #23 | #30 | |

GSDMD-CT or fragments thereof (chemically synthesized 20 polypeptide epitope sequences, from polypeptide #1 to polypeptide #20) were diluted to 2 ug/ml with PBS and 100 ul was pipetted into a 96-well plate using a sample applicator and incubated overnight at 4°C. At the end of incubation, the excess PBS was discarded from the 96-well plate, and 100 ul of PBS containing 1% casein was added to the 96-well plate and incubated at 37°C for 2 h. A sample applicator was used to pipet 100 ul of culture supernatant of hybridoma cell line (consistent with the numbering of monoclonal antibody in Tables 1-2, and have been deposited), Sp2/0 cell culture supernatant (PBS), and dilute 1000-fold positive serum to respectively add into a 96-well plate coated with TSP1-BSA, and the mixture was incubated at 37°C for 2 h. After washing three times, secondary antibodies were added and incubated for 1 h at 37°C. Washing three times, the ELISA plate was pat dried, 100 ul TMB developer was added to incubate 15 min at room temperature, then the stop solution was added to stop the development, and the absorbance value was read at 450 nm by a microplate reader.

### 2. Antibody-specific detection

The specificity of the antibodies as previously described was demonstrated by the reactivity of Specific Peptide (SEQ ID NO.4: GVPAEGAFTEDFQGLR) and Elongated Peptide (SEQ ID NO.5: TDGVPAEGAFTEDFQGLR) with antibodies. The experimental procedures were as follows:

(1) Coating antigen: Specific Peptide and Elongated Peptide were respectively diluted with the coating solution, and coated at 2 ug/ml, 100 ul/well at 4°C overnight; washed three times with the washing solution, 350 ul/well, and pat dried. (2) Blocking: 200 ul/well blocking solution, incubated at 37°C for 2 h; washed three times with the washing solution, 350 ul/well, and pat dried. (3) The antibody (such as #25, #44, or #50) supernatant medium in Table 1 was diluted by 100 times with antibody diluent, 100 ul/well, and the blank was 100 ul antibody dilution, and they were incubated at 37°C for 1 h; washed three times with the washing solution, 350 ul/well, and pat dried. (4) The secondary antibody was diluted with antibody diluent at a ratio of 1:10000, 100 ul/well, incubated at 37°C for 1 h; washed three times with the washing solution, 350 ul/well, and pat dried. (5) Color development: 100 ul/well developer was added, incubating 10 min at room temperature. (6) Termination, reading: 100 ul/well stop solution was added, reading at OD 450 nm.

Results: The three antibodies (#25, #44, or #50) had higher reactive OD values with Specific Peptide and lower OD values with Elongated Peptide, and were considered to be non-reactive with Elongated Peptide and specifically reactive with Specific Peptide (as shown in FIG. 4). As can be seen, all three antibodies are highly specific for GSDMD-CT, i.e. the N-terminal of SEQ ID NO. 4 is augmented with only two amino acids T and D at the end of its adjacent GSDMD-NT, which are no longer recognized by the antibody as previously described.

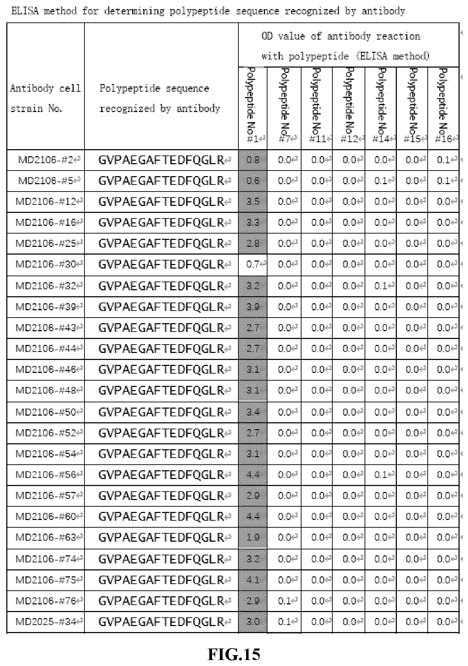

In addition, other antibodies were detected for specificity to multiple GSDMD-CT fragments (from polypeptide #1 to polypeptide #20) in a similar method, with some of the results shown in FIG. 15. It can be seen that for the GSDMD-CT fragment SEQ ID NO. 4 (polypeptide number #1), the specificity of multiple monoclonal antibodies is good, e.g. MD2106 #50. At the same time, GSDMD-CT fragment SEQ ID NO. 4 (polypeptide No. #1) is a very good target or antigen, can produce a variety of high-quality monoclonal antibodies, and can stably bind to a variety of antibodies, which can be widely used in a variety of detection protocols.

Wherein, the hybridoma cell line that produces MD2106 #50 is named MD2106-50#, and is deposited in the China General Microbiological Culture Collection Center (CGMCC), No. 3, No. 1 Beichen West Road, Chaoyang District, Beijing, and the accession number is CGMCC No. 45333. The specific information is: China General Microbiological Culture Collection Center, China Committee for Culture Collection of Microorganisms, P.O. Box 2714, Beijing, China, Postal Code: 100080, CGMCC No. 45333, Date of Deposit: November 10, 2022.

The MD2106 #50 sequence is as follows:
Heavy chain (type: IgG1) variable region amino acid sequence: 
QVQLQQPGTELVKPGASVKLSCKASGYTFTNYWIHWVKQRPGQGLEWIGNTK
PSNGDIHYNEKFRSKATLTVDKSSSTAYIQLSSLTSEDSAVYFCAVGYYWGQGTTLTVSS
(SEQ ID NO.1).
 
HCDR1: GYTFTNYW (SEQ ID NO.6); HCDR2: TKPSNGDI (SEQ ID NO.7);
HCDR3: AVGYY (SEQ ID NO.8).
Light Chains (Type: kapa) variable region amino acid sequence: 
DIVMTQSPSSLTVTAGEKVTMSCKSSQSLLNSGNQKNYLTWYQQKPGQPPKMLI
YWAYNRDSGVPDRFTGSGSGADFTLTISSVQAEDLAVYYCQNDYSYPLTFGAGTKLEL
(SEQ ID NO.2).
LCDR1: QSLLNSGNQKNY (SEQ ID NO.9); LCDR2: WAY; LCDR3: QNDYSYPLT (SEQ ID NO.10).

### 3. Antibody competitive inhibition experiment

The protein coating amount and antibody dilution concentration were preliminarily determined by checkerboard titration, and the experimental procedure was as follows:
(1) Coating antigen: The GSDMD-CT expressed protein was diluted with coating solution and coated at 2 ug/ml, 1 ug/ml, 0.5 ug/ml, 0.25 ug/ml, 100 ul/well overnight at 4°C; washed three times with the washing solution, 350 ul/well, and pat dried. (2) Blocking: 200 ul/well blocking solution, incubated at 37°C for 2 h; washed three times with the washing solution, 350 ul/well, and pat dried. (3) 1 ug/mL of #25, #44, and #50 antibodies was diluted 1,000 times with antibody diluent, then diluted with a 2-fold gradient, 100 µl/well, and blank was 100 µl of antibody diluent, and the antibodies were incubated at 37°C for 1 h; washed three times with the washing solution, 350 ul/well, and pat dried. (4) The secondary antibody was diluted with antibody diluent at a ratio of 1:10000, 100 ul/well, incubated at 37°C for 1 h; washed three times with the washing solution, 350 ul/well, and pat dried. (5) Color development: 100 ul/well developer was added, incubating 10 min at room temperature. (6) Termination, reading: 100 ul/well stop solution was added, reading at OD 450 nm. (7) The dose curve of coating protein relative to antibody was determined. The coating amount of 0.25 ug/ml was selected, and a 2-fold gradient dilution of the antibody was used for full curve fitting. The antibody dilution concentration of EC80 was selected as the antibody reaction concentration of the competition method. (8) The GSDMD-CT expressed protein was curve-fitted at a 2-fold dilution of 1000 ng/ml and the IC50 was calculated to determine the optimal antibody. (9) 100 ug/ml GSDMD-CT protein standard solution was prepared, and 1000 ng/ml, 500 ng/ml, 250 ng/ml, 125 ng/ml, 62.5 ng/ml, 31.25 ng/ml, 15.625 ng/ml, 7.813 ng/ml, 3.906 ng/ml, 1.953 ng/ml, 0.977 ng/ml, 0.488 ng/ml and 0 ng/ml GSDMD-CT sample solution were respectively prepared by stepwise dilution method. (10) 50 ul of sample solutions of different concentrations were added to 50 ul of diluted antibody, and then 100 ul of the mixture was added to the microplate reader plate and incubated for 1 h at 37°C.

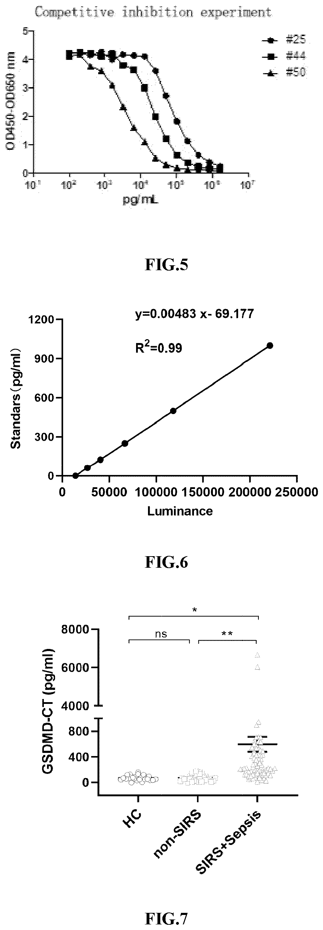

The results are shown in FIG. 5 and show that: antibodies specific for the N-terminal of GSDMD-CT, e.g. #25, #44, #50, can be prepared as immunosuppressive kits to specifically detect GSDMD-CT, e.g. for use in the assays of Examples 8-12, or can be used to detect inflammation-related diseases.

### Example 4 Subclass identification

The Ig capture antibody was diluted to 0.5 ug/ml using a PBS and 100 ul was pipetted into a 96-well plate using a sample applicator and incubated overnight at 4°C. At the end of incubation, the excess PBS was discarded from the 96-well plate, and 100 ul of PBS containing 1% casein was added to the 96-well plate and incubated at 37°C for 2 h. 100 ul of hybridoma culture supernatant was pipetted with a sample applicator and incubated in an incubator at 37°C for 2 h. After completion of the reaction, the 96-well plate was washed three times with a plate washer, and 100 ul of kappa, lambda chain diluted by 10000 times with PBS or HRP-labeled antibody diluted by 20000 was added thereto and incubated for 1 h at 37°C. Washing three times, 100 ul TMB developer was added with a sample applicator, and incubated for 15 min at room temperature, 50 ul stop solution was added to stop color development, and the absorbance value at 450 nm was read with a microplate reader.

### Example 5 Screening of paired detection antibodies (ELISA primary screening)

All monoclonal antibodies against GSDMD-CT or fragments thereof were diluted to 2 ug/ml with PBS, and 100 ul was pipetted into a 96-well plate using a sample applicator, and then the 96-well plate was placed in a 4°C cold room for overnight incubation. At the end of incubation, the excess PBS was discarded from the 96-well plate, 100 ul of PBS containing 1% casein was added to the 96-well plate, and the 96-well plate was placed in a 37°C incubator for incubation for 2 h. Then antigen with different concentrations of GSDMD-CT or fragments thereof was added and incubated at 37°C for 1h, washing three times after the reaction was completed, HRP (or ALP) labeled antibody against GSDMD-CT or fragments thereof (labeled with Label Easy rapid HRP or ALP coupling kit) 100 ul were added and incubated at 37°C for 1 h, washing three times, TMB was added and incubated at room temperature for 15 min, then stop solution was added to perform color comparison at wavelength of 450-630 nm, and the optimal antibody paired combination was selected. The best pair selected will be applied to the ELISA kit.

### Example 6 Screening of paired detection antibodies (magnetic particle chemiluminescence optimized screening)

Carboxy magnetic beads and anti-FITC monoclonal antibodies were EDC coupled according to methods known to those skilled in the art, FITC was labeled with the coated antibody initially screened with ELISA as described above, and after removal of free FITC, the labeling efficiency of the antibody and the concentration of the labeled antibody were determined. Alternatively, carboxyl magnetic beads and coated antibodies pre-screened with ELISA as described above might be subjected to EDC coupling according to methods known to those skilled in the art. The best pair selected was applied to the magnetic particle chemiluminescence kit. The detection antibody was coupled to the acridinium ester or alkaline phosphatase according to methods known to those skilled in the art.

### Example 7 Immunoassays

According to the setting requirements of SMART 500H or SMART 500S chemiluminescence instrument, alkaline phosphatase-coupled or acridinium ester-coupled detection antibody, 50 ul of plasma or standard substance and magnetic beads coupled with antibody were added into the reaction cup, incubated for 10 min, washed for 2 min, the procedure was repeated once, and the substrate was added for a reaction for 1 min. The concentration of the sample was determined with the software (spline fitting).

### Example 8 Determination of the concentration of GSDMD-CT or a fragment thereof

Antibodies against GSDMD-CT or a fragment thereof for detection of the substrate were used in Sandwich immunoassays. Concentrations above 10 pg/m of immunoreactivity of GSDMD-CT or a fragment thereof in plasma or serum could be quantitatively determined using the assay.

### Example 9 Concentration of immunoreactivity of GSDMD-CT or a fragment thereof in healthy individuals and in certain disease states

Serum and plasma from healthy individuals and patients suffering from a variety of diseases including infectious inflammation, sepsis, etc. were analyzed to determine the immunoreactivity of GSDMD-CT or fragments thereof. The immunoreactivity of GSDMD-CT or a fragment thereof was surprisingly increased in the disease state compared to healthy individuals. Specifically, GSDMD-CT concentration changes in plasma were very significant, i.e. about a 10-fold increase, in patients with sepsis (median 150, 5 pg/m) and myocardial infarction (median 129, 5 pg/ml), and about a 35-fold increase in patients with elevated arterial blood pressure (median 459, 5 pg/ml). The content of GSDMD-CT or fragments thereof measured in CHF patients correlated well with the severity of the disease.

### Example 10 Stability of immunoreactivity of GSDMD-CT or a fragment thereof

The expressed GSDMD-CT or fragments thereof were added into a protein-stabilizing buffer to prepare the calibrators and controls. The stability of immunoreactivity of GSDMD-CT or fragments thereof in calibrators and controls was tested, and the stability of GSDMD-CT or fragments thereof in calibrators and controls was shown in Table 3. As a result, the immunoreactivity of GSDMD-CT or a fragment thereof was found to be very stable.

**Table 3 Stability of calibrators and controls GSDMD-CT or fragments thereof**

| Samples | Storage (days/temperature) | Recovery rate |
|---|---|---|
| Control I (n = 3) | 1d/4°C | 98.0% |
| Control I (n = 3) | 180d/4°C | 99.3% |
| Control I (n = 3) | 1d/37°C | 96.1% |
| Control I (n = 3) | 14d/37°C | 103.4% |
| Control II (n = 3) | 1d/4°C | 102.6% |
| Control II (n = 3) | 180d/4°C | 101.6% |
| Control II (n = 3) | 1d/37°C | 98.9% |
| Control II (n = 3) | 14d/37°C | 97.3% |

### Example 11 Clinical application

### I. Materials

Plasma: The plasma samples used in the experiments were obtained from healthy persons and hospitalized patients. A total of 136 plasma samples including 24 healthy control samples (HC) and 31 samples of non-systemic inflammatory response syndrome (non-SIRS), 21 samples of systemic inflammatory response syndrome SIRS) and 60 samples of systemic infection (Sepsis) were collected in this study.

Enrollment criteria: Diagnosis according to the diagnostic criteria of the American Society of Thoracic Surgeons and the Consensus Conference on Critical Care [1]: SIRS: have the following two or more items, (1) body temperature < 36°C or > 38°C; (2) > 20 breaths/min or PCO 2 < 32 mm Hg; (3) Heart rate > 90 beats/min; (4) WBC > 12 × 10⁹/L or < 4 × 10⁹/L. Non-SIRS does not meet the diagnostic criteria for SIRS. Sepsis: Positive bacterial culture. A healthy control group (HC) was an individual who had undergone a comprehensive health assessment during a health examination. Plasma samples were transported on ice, aliquoted, and stored in a -80°C refrigerator for future use.

### II. Statistical processing

The data were processed by SPSS 22.0 software. Metrological data were analyzed by Student t or Mann-Whitney rank sum test to compare the differences between the two groups. The experiment was repeated three times and the results were statistically significant with P<0.05 expressed as mean (X) +-standard error of SEM.

### III. Results

3.1 Establishment of the standard curve: The GSDM-CT standard was diluted with PBS to 0, 62.5, 125, 250, 500, and 1000 pg/ml, and detected with the GSDMD-CT chemiluminescence detection system. Each concentration was tested 3 times. The standard curve was drawn, and the equation y = 0.00483 x -69.177 (R2 = 0.99) was obtained through curve fitting. The concentration of GSDMD-CT in plasma was calculated according to the equation (as shown in FIG. 6).

3.2 Plasma GSDMD-CT expression level was significantly increased in patients with inflammation: Analysis of plasma GSDMD-CT levels in 24 healthy controls, 21 non-inflammatory patients, and 91 inflammatory patients using the antibodies of the present application (e.g. antibodies #25, #44, and #50) found that GSDMD-CT concentrations were 71.0 ± 8.3 pg/ml in the healthy group, 72.3 ± 9.5 pg/ml in the non-inflammatory group, and 596.8 ± 109.7 pg/ml in the inflammatory group. There was no significant difference in plasma GSDMD-CT between the non-inflammatory group and the healthy control group (P>0.05). The expression level of GSDMD-CT in the inflammatory group was significantly higher than that in the healthy group and the non-inflammatory group (P<0.05), and the results were shown in FIG. 7.

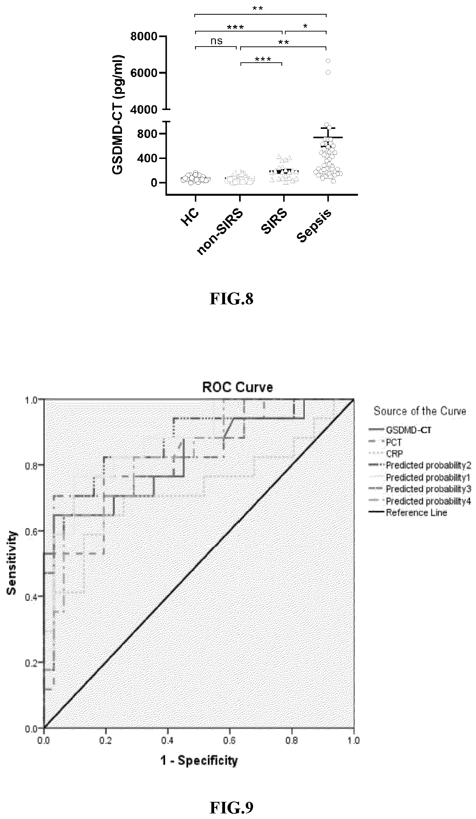

Further analysis, inflammation was divided into two groups: systemic inflammatory response syndrome (SIRS) and infectious inflammation (Sepsis), and the two groups were compared respectively. The plasma GSDMD-CT concentration was 186.3 ± 27.6 pg/ml in the SIRS group and 740.5 ± 152.6 pg/ml in the Sepsis group. The levels of plasma GSDMD-CT in the SIRS group and Sepsis group were significantly higher than those in the healthy group (HC) and non-inflammatory group (non-SIRS) (P<0.05), and the expression level of plasma GSDMD-CT in Sepsis group was significantly higher than that in SIRS group (P<0.05). The results were shown in FIG. 8.

3.3 Expression level of GSDMD-CT and general clinical characteristics of patients: Further, we analyzed the relationship between the general characteristics of 112 clinical cases and GSDMD-CT, and found that the expression level of plasma GSDMD-CT was not related to the patient's age and gender (P>0.05). The results were shown in Table 4.

**Table 4. General characteristics of GSDMD-CT vs. non-SIRS, SIRS, and Sepsis case**

| Variable | Tota | GSDMD-CT (pg/mL) | | P value |
|---|---|---|---|---|
| | | mean±SEM | Rang | |
| Gender | 112 | | | P>0.05 |
| Male | 60 | 334.2±102.0 | 9.1-6033.9 | |
| Femal | 52 | 587.0±144.3 | 0.0000-6666.7 | |
| Age | 112 | | | P>0.05 |
| ≥60 | 67 | 453.8±110.7 | 0.0000-6666.7 | |
| <60 | 45 | 448.3±141.4 | 12.1-6033.9 | |
| Diagnosis | 112 | | | |
| non-SIRS | 31 | 72.3±9.5 | 0.0000-176.1 | P>0.05 |
| SIRS | 21 | 186.3±27.6 | 12.3-432.7 | P<0.01 |
| Sepsis | 60 | 740.5±152.6 | 26.0-6666.7 | P<0.01 |

3.4 Plasma GSDMD-CT is a potential biomarker for SIRS: the ROC curve was used to analyze the diagnostic efficacy of plasma GSDMD-CT for SIRS. The area under the ROC curve of plasma GSDMD-CT for SIRS diagnosis was 0.824, which was better than that of procalcitonin (PCT) 0.811 and C-reactive protein (CRP) 0.723 for SIRS. The area under the ROC curve for the diagnostic efficacy of GSDMD-CT combined with PCT and CRP for SIRS was 0.875, which was superior to the diagnostic efficacy of GSDMD-CT combined with PCT (0.861), GSDMD-CT combined with CRP (0.841), and PCT combined with CRP (0.833) for SIRS. The results as described above indicated that plasma GSDMD-CT was a novel marker for predicting SIRS, and the results were shown in FIG. 9 and Table 5.

**Table 5 Area under the curve**

| Test Result Variable(s) | Area | Std. Error^{a} | Asymptotic Sig.^{b} | Asymptotic 95% Confidence Interval | |
|---|---|---|---|---|---|
| | | | | Lower Bound | Upper Bound |
| GSDMD-CT | .824 | .068 | .000 | .691 | .958 |
| PCT | .811 | .066 | .000 | .682 | .940 |
| CRP | .723 | .086 | .011 | .554 | .892 |
| Predicted probability 2 | .861 | .059 | .000 | .745 | .978 |
| Predicted probability 1 | .875 | .053 | .000 | .771 | .978 |
| Predicted probability 3 | .841 | .065 | .000 | .714 | .967 |
| Predicted probability 4 | .833 | .061 | .000 | .714 | .952 |

The test result variable(s): GSDMD-CT, PCT has at least one tie between the positive actual state group and the negative actual state group. Statistics may be biased.
a. Under the nonparametric assumption
b. Null hypothesis: true area = 0.5

Wherein:

| | |
|---|---|
| Predicted probability 2 | GSDMD-CT in combination with PCT |
| Predicted probability 1 | GSDMD-CT in combination with PCT, CRP |
| Predicted probability 3 | GSDMD-CT in combination with CRP |
| Predicted probability 4 | PCT in combination with CRP |

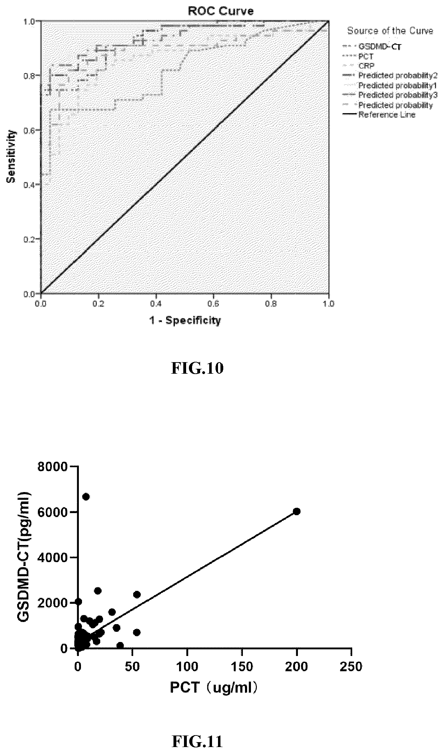

3.5 Plasma GSDMD-CT is a potential biomarker for Sepsis: the ROC curve was used to analyze the diagnostic efficacy of plasma GSDMD-CT for SIRS. The area under the ROC curve of plasma GSDMD-CT for Sepsis diagnosis was 0.928, which was better than that of procalcitonin (PCT) 0.820 and C-reactive protein (CRP) 0.860 for Sepsis. The area under the ROC curve for the diagnostic efficacy of GSDMD-CT combined with PCT and CRP for Sepsis was 0.946, which was superior to that of GSDMD-CT combined with PCT (0.940), GSDMD-CT combined with CRP (0.944), and PCT combined with CRP (0.889) for Sepsis. The results as described above indicated that plasma GSDMD-CT was a new marker for predicting Sepsis, and the results were shown in FIG. 10 and Table 6.

**Table 6 Area under the curve**

| Test Result Variable(s) | Area | Std. Error^{a} | Asymptotic Sig.^{b} | Asymptotic 95% Confidence Interval | |
|---|---|---|---|---|---|
| | | | | Lower Bound | Upper Bound |
| GSDMD-CT | .928 | .026 | .000 | .878 | .979 |
| PCT | .820 | .044 | .000 | .733 | .906 |
| CRP | .860 | .040 | .000 | .781 | .939 |
| Predicted probability 2 | .940 | .024 | .000 | .893 | .986 |
| Predicted probability 1 | .946 | .022 | .000 | .903 | .990 |
| Predicted probability 3 | .944 | .022 | .000 | .900 | .988 |
| Predicted probability 4 | .889 | .036 | .000 | .818 | .961 |

The test result variable(s): PCT has at least one tie between the positive actual state group and the negative actual state group. Statistics may be biased.
a. Under the nonparametric assumption
b. Null hypothesis: true area = 0.5

| | |
|---|---|
| Predicted probability 2 | GSDMD-CT in combination with PCT |
| Predicted probability 1 | GSDMD-CT in combination with PCT, CRP |
| Predicted probability 3 | GSDMD-CT in combination with CRP |
| Predicted probability 4 | PCT in combination with CRP |

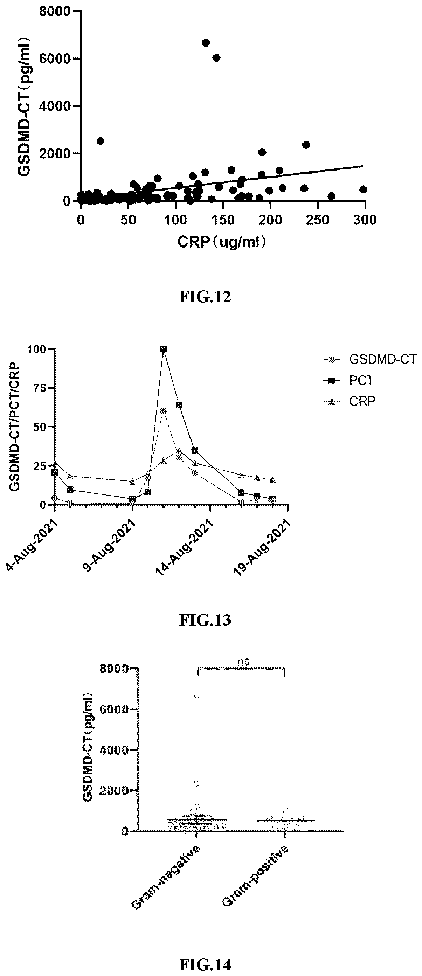

3.6 Plasma GSDMD-CT or fragments thereof were positively correlated with the expression of PCT. We analyzed the correlation between plasma GSDMD-CT and the expression levels of PCT and CRP in patients using the binary regression method and found that GSDMD-CT was significantly positively correlated with the expression of PCT (r = 0.65), but weakly correlated with the expression of CRP (r = 0.32). The results were shown in FIG. 11 and FIG. 12. We further selected a case for continuous monitoring and found that the changes of GSDMD-CT were consistent with those of CRP and PCT, and in severe cases, their concentration changes were more significant than those of CRP and PCT (as shown in FIG. 13).

In conclusion, GSDMD-CT is a new marker to judge the inflammatory state of patients.

### Example 12 Comparison of GSDMD-CT concentration content in patient samples infected with Gram-negative bacteria and Gram-positive bacteria

The present inventors tested the concentration content of GSDMD-CT in the samples of patients with Gram-negative and Gram-positive bacterial infections and found that GSDMD-CT could detect both Gram-negative and Gram-positive bacterial infections, and both types of bacterial infections could cause an increase in GSDMD-CT (as shown in FIG. 14). Therefore, GSDMD-CT can be used as a marker of bacterial infection.

## Claims

1. A marker, wherein the marker is GSDMD-CT or a fragment thereof, a modified variant of GSDMD-CT or a fragment thereof, and/or a derivative of GSDMD-CT or a fragment thereof.

2. The marker according to claim 1, **characterized in that** the sequence of wherein the GSDMD-CT is as shown in SEQ ID NO. 3; or the sequence of the fragment is as shown in SEQ ID NO. 4.

3. A nucleic acid encoding the marker according to claim 1 or 2.

4. A biological material comprising the marker according to any one of claims 1 to 3 or the nucleic acid according to claim 4, wherein the material is one or more of an expression cassette, a recombinant vector, a recombinant microorganism, or a transgenic cell line.

5. A ligand binding to the marker according to claim 1 or 2, the nucleic acid according to claim 3, and/or the biological material according to claim 4.

6. The ligand according to claim 5, wherein the ligand comprising a nucleic acid aptamer, a peptide aptamer, a peptibody, a mimetic, a phage, an inhibitor, a compound, and/or an antibody.

7. The ligand according to claim 6, wherein the antibody comprises a Nanobody, a monospecific antibody, a polyclonal antibody, and/or an antigen-binding fragment of an antibody; for example, a monoclonal antibody prepared by immunizing a mouse with the polypeptide as shown in SEQ ID NO. 4.

8. The ligand according to any one of claims 5 to 8, wherein the ligand bears a recognizable label; preferably, wherein the label comprises a dye, an epitope tag, a fluorescent moiety, a luminescent moiety, a chemiluminescent moiety, an enzyme label, a magnetic label, a paramagnetic label, a contrast agent, a nanoparticle, a radioisotope, biotin, streptavidin, and/or a quencher.

9. A hybridoma cell line, **characterized by** being designated as MD2106-50# and being deposited under the accession number CGMCC No. 45333.

10. A monoclonal antibody #50 or an antigen-binding fragment thereof, **characterized by** comprising antigen complementarity determining regions of a heavy chain variable region, CDR1, CDR2, and CDR3, and antigen complementarity determining regions of a light chain variable region, CDR1, CDR2, and CDR3;
amino acid sequences of the antigen complementarity determining regions CDR1, CDR2, and CDR3 of the heavy chain variable region are SEQ ID NO. 6: GYTFTNYW, SEQ ID NO. 7: TKPSNGDI, and SEQ ID NO. 8: AVGYY respectively;
amino acid sequences of the antigen complementarity determining regions CDR1, CDR2, and CDR3 of the light chain variable region are SEQ ID NO. 9: QSLLNSGNQKNY, WAY, and SEQ ID NO.10: QNDYSYPLT respectively.

11. The monoclonal antibody #50 or the antigen-binding fragment thereof according to claim 10, wherein the antigen-binding fragment includes an Fab fragment, an Fab', an F(ab') 2 fragment, an Fd fragment; an Fv fragment, a dAb fragment, a single chain scFv, a single chain scFv-Fc fragment, or a single chain antibody ScAb.

12. A cell line producing the monoclonal antibody #50 or the antigen-binding fragment thereof according to claim 10 or 11.

13. A nucleic acid molecule, **characterized by** encoding the monoclonal antibody #50 or the antigen-binding fragment thereof according to claim 10 or 11.

14. An expression cassette, a recombinant vector, or a recombinant microorganism, **characterized by** comprising the nucleic acid molecule according to claim 13.

15. A kit, or a composition, or a system, or a device, **characterized by** comprising the marker according to claim 1 or 2, the nucleic acid according to claim 3, the biological material according to claim 4, the ligand according to any one of claims 5 to 8, the cell line according to claim 9, the monoclonal antibody #50 or the antigen-binding fragment thereof according to claim 10 or 11, the cell line according to claim 12, the nucleic acid molecule according to claim 13, or the expression cassette, a recombinant vector, or a recombinant microorganism according to claim 14.

16. Use of a "substance or system", wherein the "substance or system" comprises: the marker according to claim 1 or 2, the nucleic acid according to claim 3, the biological material according to claim 4, the ligand according to any one of claims 5 to 8, the cell line according to claim 9, the monoclonal antibody #50 or the antigen-binding fragment thereof according to claim 10 or 11, the cell line according to claim 12, the nucleic acid molecule according to claim 13, the expression cassette, a recombinant vector, or a recombinant microorganism according to claim 14, and/or the kit, a composition, a system, or a device according to claim 15;
wherein the use is one of the following:
(a) for determining the degree of pyroptosis, or for preparing a preparation for determining the degree of pyroptosis;
(b) for inhibiting pyroptosis, or for preparing a preparation for inhibiting pyroptosis;
(c) for preventing or diagnosing inflammation-related diseases, or for preparing a preparation for preventing or diagnosing inflammation-related diseases;
(d) for treating inflammation-related diseases, or for preparing a drug for treating inflammation-related diseases;
(e) for assessing the severity of inflammation-related diseases or the prognosis of a patient suffering from inflammation-related diseases, or for preparing a preparation for assessing the severity of inflammation-related diseases or the prognosis of a patient suffering from inflammation-related diseases; and
(f) for monitoring the progression of inflammation-related diseases, or for preparing a preparation for monitoring the progression of inflammation-related diseases.

17. A method of screening for a drug, a compound, a composition or a preparation for detecting or inhibiting the degree of pyroptosis, for preventing or diagnosing inflammation-related diseases, for treating inflammation-related diseases, for assessing the severity of inflammation-related diseases or the prognosis of a patient suffering from inflammation-related diseases, or for monitoring the progression of inflammation-related diseases, **characterized by** comprising using of the marker according to claim 1 or 2, the nucleic acid according to claim 3, the biological material according to claim 4, the ligand according to any one of claims 5 to 8, the cell line according to claim 9, the monoclonal antibody #50 or the antigen-binding fragment thereof according to claim 10 or 11, the cell line according to claim 12, the nucleic acid molecule according to claim 13, the expression cassette, a recombinant vector, or a recombinant microorganism according to claim 14, and/or the kit, a composition, a system, or a device according to claim 15.

18. A method, **characterized by** comprising using of the marker according to claim 1 or 2, the nucleic acid according to claim 3, the biological material according to claim 4, the ligand according to any one of claims 5 to 8, the cell line according to claim 9, the monoclonal antibody #50 or the antigen-binding fragment thereof according to claim 10 or 11, the cell line according to claim 12, the nucleic acid molecule according to claim 13, the expression cassette, a recombinant vector, or a recombinant microorganism according to claim 14, and/or the kit, a composition, a system, or a device according to claim 15; wherein the method is one of the following:
(a) determining the degree of pyroptosis, or preparing a preparation for determining the degree of pyroptosis;
(b) inhibiting pyroptosis, or preparing a preparation for inhibiting pyroptosis;
(c) preventing or diagnosing inflammation-related diseases, or preparing a preparation for preventing or diagnosing inflammation-related diseases;
(d) treating inflammation-related diseases, or preparing a drug for treating inflammation-related diseases;
(e) assessing the severity of inflammation-related diseases or the prognosis of a patient suffering from inflammation-related diseases, or preparing a preparation for assessing the severity of inflammation-related diseases or preparing a preparation for assessing the prognosis of a patient suffering inflammation-related diseases; and
(f) monitoring the progression of inflammation-related diseases, or preparing a preparation for monitoring the progression of inflammation-related diseases.

19. The use according to claim 16, the method according to claim 17, or the method according to claim 18, **characterized by** comprising the steps of: (i) detecting one or more proteins or nucleic acids in the sample and quantifying the levels thereof, (ii) comparing wherein the quantified levels with the levels of the corresponding proteins or nucleic acids in a control population; wherein the protein comprises the marker according to claims 1 and 2 or the nucleic acid according to claim 3.

20. According to any one of the preceding claims, wherein the inflammation-related diseases comprise diseases caused by pyroptosis or diseases accompanied by cellular inflammatory necrosis associated with pyroptosis; preferably, wherein the inflammation-related diseases are: inflammatory diseases caused by infection, especially inflammatory diseases caused by bacterial infection, inflammatory diseases caused by viral infection, such as pneumonia, peritonitis, cholangitis, urinary system infection, cellulitis, meningitis, abscess and the like and complications of infection thereof, surgery, trauma, multiple trauma, burn, septicemia, sepsis, septic shock and the like; autoimmune diseases such as arthritis, gout, scleroderma, and the like; chronic diseases such as diabetes, chronic obstructive bronchial disease, leukemia, aplastic anemia, urinary calculi and the like; other diseases such as one or more of cancer, diabetes, kidney disease, senile dementia and the like.
